# EUROPÄISCHE PATENTSCHRIFT

(11) **EP 0 686 398 B1**
(45) Veröffentlichungstag und Bekanntmachung des Hinweises auf die Patenterteilung: **31.01.2001**
(21) Anmeldenummer: 95108859.0
(22) Anmeldetag: 08.06.1995
(51) Int. Cl.: A61K 39/205, A61K 9/00

(54) **Tierköder**
Animal bait
Appât pour animaux

(30) Priorität: 10.06.1994 DE 4420438
(43) Veröffentlichungstag der Anmeldung: 13.12.1995
(73) Patentinhaber: Altrogge-Holding S.A., CH-8750 Glarus (CH)
(72) Erfinder: Altrogge, Hans, D-32791 Lage (DE)
(74) Vertreter: Blumbach, Kramer & Partner GbR

(56) Entgegenhaltungen:
- EP-A- 0 240 826
- DE-A- 4 233 625
- US-A- 4 014 991
- US-A- 4 650 673

## Beschreibung

Die Erfindung betrifft einen Tierköder zur Vorbeugung gegen und/oder zur Behandlung von Krankheiten bei wildlebenden Tieren mit einem oder mehreren pharmazeutischen Wirkstoff(en) mit einem speziellen Umhüllungsmaterial. Die Erfindung betrifft außerdem ein Verfahren zur Herstellung eines derartigen Tierköders.

Letztlich betrifft die Erfindung auch die Verwendung eines derartigen Tierköders zur Vorbeugung und/oder Bekämpfung von Krankheiten in freier Wildbahn lebender Tiere.

Die Verabreichung von Arzneimitteln an in freier Wildbahn lebende Tiere stellt ein seit langer Zeit nicht befriedigend gelöstes Problem dar. Dies resultiert einerseits daraus, daß pharmazeutische Wirkstoffe in geeigneter Formulierung entwickelt werden mußten, um Tieren ohne unmittelbaren Kontakt mit den Menschen pharmazeutische Wirkstoffe zu verabreichen, andererseits jedoch Verabreichungswege gefunden werden mußten, um in freier Wildbahn lebende Tiere zur Aufnahme der entsprechenden pharmazeutischen Wirkstoffe zu bewegen. Ein Beispiel hierfür stellt die Bekämpfung der Wildtollwut in Europa und in den Vereinigten Staaten von Amerika dar. Einen zusammenfassenden Bericht hierüber liefert der Artikel "W. G. Winkler und K. Boegel: Bekämpfung der Wildtollwut; in: Spektrum der Wissenschaft, August 1992, S. 85 bis 93".

Für den speziellen Zweck der Bekämpfung der Wildtollwut bei Füchsen wurden - nach allzu gefährlich empfundenen Versuchen der Impfung der Füchse mit selbstauslösenden Impffallen - sogenannte "Wurstköder" entwickelt. Derartige Wurstköder sind beispielsweise in der US-A 4,014,991 sowie in "Develop. Biol. Standard., Bd. 33, S. 417 bis 423 (1976); G. M. Baer: The oral rabies immunisation of foxes and dogs with sausage baits" offenbart. Derartige Wurstköder bestanden aus einer üblichen geräucherten Wurst o. ä., in deren Mitte ein Kunststoffröhrchen mit einem Impfstoff eingebracht war. Die Füchse sollten in die so präparierten Köder beißen und damit auch das darin enthaltene Kunststoffröhrchen durchlöchern oder zerbrechen. Der oral aufnehmbare Tollwut-Impfstoff benetzte dann ihren Fang bzw. ihre Zunge, so daß der Impfstoff über die Schleimhaut des Mauls aufgenommen und wirksam werden konnte.

In "Spektrum der Wissenschaft, a.a.O." wird auch von mit Impfstoff gefüllten Plastikbehältern präparierten Hühnerköpfen als Köder für die Verabreichung von Arzneimitteln, insbesondere von Tollwut-Impfstoffen, berichtet. Diese Köder wiesen jedoch in der Praxis den Nachteil auf, daß der in den Hühnerschnabel eingeführte Impfstoffbehälter beim (artgerechten) Spielen des Fuchses mit dem Köder häufig verlorenging und damit der angestrebte Zweck, den Fuchs oral gegen Tollwut zu impfen, nicht erreicht werden konnte.

Die bis dahin bekannten Köder wiesen darüber hinaus den Nachteil auf, daß sie nicht nur in Handarbeit hergestellt werden mußten und sich daher kaum für die Massenproduktion eigneten, sondern auch per Hand ausgelegt werden mußten. Letzteres war jedoch nicht nur äußerst mühsam, sondern erhöhte auch die mit der Auslegung verbundenen Unsicherheitsfaktoren. Vor allem war dies der Kontakt eines Menschen mit dem Ködermaterial, der zur Folge hatte, daß der Köder von dem Wildtier nicht mehr angenommen wurde.

In der US-A 4 014 991 wird ein Impfstoff und ein Verfahren zum Schutz wildlebender Fleischfresser, insbesondere Füchse, gegen Tollwut, durch Aussetzung und orale Verabreichung einer immunisierenden Dosis eines verdünnten flüssigen Anti-Tollwutimpfstoffes offenbart, wobei der Impfstoff in einem durchbeißbaren hydrophoben Kunststoffbehälter oder einer Ummantelung enthalten ist und der Behälter mit einem Fleischköder für den Fleischfresser umgeben ist.

In der EP-A 0 240 826 wird ein weiterer Köder für Tiere mit einem pharmazeutischen Wirkstoff, beispielsweise zur Tollwutimpfung, offenbart. Darin ist der Wirkstoff in einer fetthaltigen Trägersubstanz enthalten, die außerdem einen spezifischen Lockstoff enthält. Die Fettkomponente der Trägersubstanz weist einen Schmelzpunkt zwischen 20 und 60° C auf. Ein derartiger Köder wird hergestellt unter Verflüssigung der Fettkomponente, Zumischen gewisser Zuschlagsstoffe, die zur Stabilisierung der Formhaltigkeit des Köders dienen sollen, und Einfüllen des flüssigen Gemischs in eine (gegebenenfalls auch der weiteren Handhabung dienende) Form. In das abgekühlte Gemisch wird eine in Form einer Blisterpackung vorliegende separate Verpackungseinheit mit dem pharmazeutischen Wirkstoff eingelegt, speziell einem flüssigen Tollwut-Impfstoff. Die Form wird danach mit dem unter die Aktivierungstemperatur des verwendeten Wirkstoffs abgekühlte pastöse Trägermasse aufgefüllt, bis die Wirkstoffeinheit vollständig mit der Trägermasse bedeckt ist. So wird ein (im wesentlichen scheibenförmiger) Köder hergestellt, dessen Trägermasse aus tierischen Fetten und Proteinkomponenten wie beispielsweise für Futterzwecke hergestellten Tiermehlen und/oder Fleischmehlen und einem für Füchse spezifischen Lockstoff besteht, beispielsweise Fischmehl.

Nachteil der in der EP-A 0 240 826 beschriebenen Tierköder zur Tollwutimpfung war jedoch ihre Klebrigkeit, die eine praktische Handhabung außerordentlich erschwerte. Darüber hinaus konnte nicht sichergestellt werden, daß bei der Ausbringung der Köder, beispielsweise beim Abwurf aus Flugzeugen aus größerer Höhe, das den Köderkörper bildende Material ausreichend stabil war und nicht zerbrach oder zumindest anbrach und damit die darin enthaltene Wirkstoffeinheit entweder den Umgebungseinflüssen aussetzte, oder den gebrochenen Köder für die Füchse so wenig attraktiv (oder in Folge der blinkenden Blisterpackung sogar abschreckend) machte, daß er nicht in ausreichender Weise angenommen wurde. Zum anderen bestand eine gewisse Gefahr, daß der aus Flugzeugen abgeworfene Köderkörper unbeabsichtigt einen sich im Abwufgebiet aufhaltenden Menschen traf und möglicherweise verletzte oder zumindest mit dem unangenehm riechenden Ködermaterial beschmutzte.

Darüber hinaus war die Herstellung eines derartigen Köders insofern problematisch, als durch die bei erhöhter Temperatur zu verarbeitende Trägermasse die Aktivität des pharmazeutischen Wirkstoffs, beispielsweise des flüssigen Tollwutvirus-Impfstoffs abgesenkt wurde. Darüber hinaus bildete sich bei der Abkühlung der Trägermasse um die Wirkstoffeinheit Kondenswasser, das häufig zu einem Bruch der Trägermasse führte. Die genannten Probleme konnten bisher nicht vollständig beseitigt werden.

In der DE-A 4 233 625 wurde ein Tierköder mit einem oder mehreren pharmazeutischen Wirkstoffen, einem oder mehreren festen Polymeren tierischer oder pflanzlicher Herkunft als Trägersubstanzen einem für die zu ködernde Tierart spezifischen Lockmittel und einem oder mehreren in der Trägersubstanz in fester oder flüssiger Form enthaltenen pharmazeutischen Wirkstoffen offenbart. Der genannte Tierköder bezog seine Formstabilität aus dem bzw. den ausgehärteten Polymer(en) tierischer oder pflanzlicher Herkunft. In einer bevorzugten Ausführungsform besteht der Köder nach dem Gebrauchsmuster G 92 13 449.1 aus einer äußeren sogenannten Kompressionshülle relativ höher Dichte und Festigkeit, die gegebenenfalls ein Bindemittel und gegen Witterungseinflüsse beständig machende Komponenten enthält, und einem pulverartigen oder granulatartigen, Trägersubstanz und einen oder mehrere pharmazeutischen Wirkstoffe umfassenden Kern relativ niedriger Dichte und Festigkeit. Der so aufgebaute Formkörper konnte die Erfordernisse hinsichtlich Stabilität und Wetterbeständigkeit weitgehend erfüllen und erwies sich auch im praktischen Test als optimal im Hinblick auf die Annahme durch in freier Wildbahn lebende Tiere. Nachteilig war jedoch, daß bei vorgegebener Impfstoffmenge, die in der Praxis etwa 1,5 bis 2 ml pro Köderkörper betragen muß, um eine ausreichende Aufnahmewahrscheinlichkeit des flüssigen Impfstoffs durch die oralen Schleimhäute des Fuches sicherzustellen, ein viel zu umfangreicher Köderkörper verwendet werden mußte, was bei der Ausbringung und praktischen Handhabung, insbesondere beim Abwurf größerer Ködermengen vom Flugzeug, hinderlich war.

Der vorliegenden Erfindung lag die Aufgabe zugrunde, einen im industriellen Maßstab herstellbaren Tierköder bereitzustellen. Dieser Köder sollte im Rahmen eines Verfahrens herstellbar sein, bei dem eine Belastung des pharmazeutischen Wirkstoffs, beispielsweise eines flüssigen Virusimpfstoffs, nicht auftritt.

Darüber hinaus sollte der Köder in einer Weise gegen Umwelteinflüsse thermisch und mechanisch stabil sein, die ihn für die Herstellung bei industriell realisierbaren Temperaturen sowie die Lagerung und Handhabung sowie die Auslegung bei üblichen Auslegungsbedingungen (-temperaturen bzw. -feuchten) geeignet macht.

Eine weitere Aufgabe der Erfindung war, einen Tierköder mit einem pharmazeutischen Wirkstoff bereitzustellen, der nicht zu einem Wirkstoffverlust des pharmazeutischen Wirkstoffs beim Herstellen führt, keine Belästigung der Umwelt, beispielsweise durch Gerüche oder die Gefahr des Treffens mit einem großvolumigen Köderkörper, mit sich bringt und unter Anwendungstemperaturen und -drücken bzw. -feuchten thermisch und mechanisch stabil ist. Eine Herstellung des Köders aus preiswert zugänglichen und unter natürlichen Bedingungen verrottbaren Ausgangsmaterialien in einfacher Weise in großindustriellem Maßstab mit hohem Automatisierungsgrad sollte gesichert sein.

Darüber hinaus war eine wesentliche Aufgabe der vorliegenden Erfindung, einen Tierköder bereitzustellen, der zur Verwendung in einem automatisierbaren Ausbringverfahren geeignet ist. Das letztgenannte Erfordernis gewinnt dadurch immer weiter an Wichtigkeit, daß zur Sicherstellung einer flächendeckenden Krankheitsbekämpfung, beispielsweise einer flächendeckenden Tollwut-Bekämpfung, eine Ausbringung größerer Ködermengen mit wirtschaftlichen und kontrollierbaren Methoden verlangt wird und auch erforderlich ist. Dabei ist die weitgehende Garantie, daß ein bestimmtes Areal, in dem Wildtiere leben, zuverlässig mit von dem Wildtier aufgenommenen Ködern versorgt ist, unverzichtbar.

Ein wesentlicher Nachteil bisher bekannter Köder, die zur Krankheitsbehandlung oder Krankheitsvorsorge, beispielsweise zur Tollwutimpfung, von wildlebenden Tieren verwendet wurden, war - mit Ausnahme der in dem deutschen Gebrauchsmuster G-92 13 449.1 beschriebenen Köder - ihre relativ problematische Handhabung, insbesondere unter Ausbringungsbedingungen. So bestanden regelmäßig Probleme mit der Klebrigkeit des Trägermaterials von aus dem Stand der Technik bekannten Ködern. Insbesondere bei erhöhten Handhabungstemperaturen klebten mehrere Köderkörper zusammen und konnten nur unter teilweiser oder vollständiger Zerstörung der Integrität des einzelnen Köderkörpers wieder voneinander getrennt werden. Dadurch gingen nicht nur größere Ködermengen bereits vor der Auslegung wieder verloren, sondern wurden auch immer wieder einzelne Köderkörper beschädigt. Die Folge war, daß die darin enthaltene Impfstoff-Verpackung ganz oder teilweise den Umwelteinflüßen ausgesetzt war. Erhebliche Verluste an Wirksamkeit des Impfstoffs einerseits und Abschreckung der Tiere durch das blinkende Metall der Impfstoff-Verpackung andererseits waren die Folge. Beides führte immer wieder zu erheblichen wirtschaftlichen Verlusten.

Darüber hinaus waren - wie vorstehend im einzelnen erläutert - die Köderkörper relativ voluminös. Dies erforderte nach der Herstellung des Köders eine enorme Lager- und Kühlkapazität. Darüber hinaus wurde dann, wenn die Köder durch Abwurf vom Flugzeug ausgebracht wurden, die Ladefähigkeit des Flugzeugs durch das erhebliche Gewicht des Köder-Körpers relativ stark eingeschränkt. Eine wirtschaftliche Ausbringung war daher insbesondere in Gebieten, in denen genügend starke Maschinen nicht zur Verfügung standen, kaum noch möglich.

Der vorliegenden Erfindung lag daher die weitere Aufgabe zugrunde, ein Verfahren zur Herstellung eines Tierköders zur Verfügung zu stellen, das in einfacher und preiswerter Weise zu dem gewünschten, hochwirksamen Köder-Körper führte und darüber hinaus eine wirtschaftliche Ausbringung der resultierenden Köder ermöglichte.

Eine weitere Aufgabe bestand darin, ein Verfahren zur Herstellung und Ausbringung der Köder bereitzustellen, das mit einfachen, wenig kostenträchtigen Verfahrensschritten auskommt und darüber hinaus eine raumsparende Lagerung, Kühlung und Ausbringung der fertigen, mit medizinischem Wirkstoff beaufschlagten Köder ermöglichte.

Die Erfindung betrifft einen Tierköder zur Vorbeugung gegen und/oder Behandlung von Krankheiten wildlebender Tiere mit einem oder mehreren pharmazeutischen Wirkstoff(en), der aus einem den/die pharmazeutische(n) Wirkstoff(e) vollständig umhüllenden, mit diesem/diesen nicht in Wechselwirkung tretenden Material besteht, das gleichzeitig so beschaffen ist, daß ein gegebenenfalls auf der Außenseite der daraus gebildeten vollständigen Umhüllung aufgebrachtes, für die zu Ködernde Tierarf spezifisches Lockmittel darauf haftet.

Die Erfindung betrifft auch ein Verfahren zur Herstellung eines einen oder mehrere pharmazeutische(n) Wirkstoff(e) enthaltenden Tierköders zur Vorbeugung gegen und/oder Behandlung von Krankheiten wildlebender Tieren, das gekennzeichnet ist durch die Schritte, daß man
(a) ein zum vollständigen Umhüllen des/der pharmazeutischen Wirkstoffe(s) geeignetes Material vorlegt und zu einer Tasche formt;
(b) die Tasche unter für den/die pharmazeutischen Wirkstoff(e) unschädlichen Bedingungen mit dem/den pharmazeutischen Wirkstoff(en) befüllt und unter Bildung einer vollständigen Umhüllung versiegelt;
(c) das so gebildete Wirkstoffpäckchen gegebenenfalls unter geeigneten Bedingungen lagert;
(d) vor der Ausbringung, bevorzugt unmittelbar vor der Ausbringung, das Wirkstoffpäckchen gegebenenfalls mit einem für die zu ködernde Wild-Tierart spezifischen Lockmittel auf der Außenseite beaufschlagt; und
(e) das so gebildete, einen oder mehrere pharmazeutische(n) Wirkstoff(e) einschließende, gegebenenfalls ein Lockmittel auf der Außenseite umfassende Wirkstoffpäckchen zur Vorbeugung gegen und/oder Behandlung von Krankheiten wildlebender Tiere auslegt.

Die Erfindung betrifft auch Tier Koder gemäß der nachgfolgenden Beschreibung zur Verwendung bei der Vorbeugung gegen und/oder Behandlung von Krankheiten wildlebender Tiere, insbesondere bei der Bekämpfung von Wildtollwut bei Füchsen.

Die Erfindung wird nachfolgend im einzelnen beschrieben. Im Rahmen der Beschreibung wird dabei wiederholt auf eine beispielhafte Ausführungsform des erfindungsgemäßen Tierköders Bezug genommen, die sich zur Verabreichung eines Tollwut-Impfstoffs an wildlebende Tiere wie beispielsweise Füchse eignet. Die Erfindung ist jedoch nicht auf diese Ausführungsform beschränkt. Vielmehr umfaßt der erfindungsgemäße Tierköder auch zur Verabreichung eines oder mehrerer anderer pharmazeutischer Wirkstoffe geeignete Tierköder. Es können dabei einzelne pharmazeutische Wirkstoffe oder eine Kombination mehrerer pharmazeutischer Wirkstoffe in dem erfindungsgemäßen Tierköder enthalten sein. So ist es auch denkbar, daß die erfindungsgemäßen Tierköder zur Verabreichung von Impfstoffen gegen Krankheiten ebenfalls geeignet sind, die bei freilebenden Wildtieren jeglicher Art auftreten. Eine besonders gute Eignung zeigen die erfindungsgemäßen Tierköder jedoch dann, wenn es sich bei dem pharmazeutischen Wirkstoff um einen Virusimpfstoff gegen Tollwut handelt. Insofern stellt der erfindungsgemäße Tierköder mit einem gegen den Tollwutvirus gerichteten Impfstoff eine bevorzugte Ausführungsform dar. Dabei können beliebige, an sich aus dem Stand der Technik bekannte Impfstoffe gegen den Tollwutvirus verwendet werden.

Beispielhaft ist ein flüssiger Tollwutvirus-Impfstoff zu nennen, der auf oralem Wege, d.h. bei Kontakt der Schleimhäute im Fang (Schnauze) oder auf der Zunge des Tieres, wirkt.

Wenn mehrere pharmazeutische Wirkstoffe miteinander kombiniert werden, dürfen sich diese hinsichtlich ihrer pharmazeutischen Wirksamkeit nicht gegenseitig beeinflussen. So ist es beispielsweise möglich, einen Wirkstoff gegen Tollwut mit pharmazeutischen Mitteln zu kombinieren, die die allgemeine Widerstandsfähigkeit des entsprechenden Tieres, beispielsweise eines Fuchses, gegenüber viralen Erkrankungen verbessern können.

Erfindungsgemäß besteht der Tierköder aus einem den/die pharmazeutische(n) Wirkstoff(e) vollständig umhüllenden, mit diesem bzw. diesen nicht in Wechselwirkung tretenden material, das gleichzeitig so beschaffen ist, daß ein gegebenenfalls auf der Außenseite der daraus gebildeten vollständigen Umhüllung aufgebrachtes, für die zu ködernde Tierart spezifisches Lockmittel darauf haftet.

Aus vorstehendem ergibt sich, daß das den mit einem oder mehreren pharmazeutischen Wirkstoff(en) umgebende Material zwei Funktionen hat: Zum einen soll das Material den/die pharmazeutischen Wirkstoff(e) vollständig umhüllen, d.h. quasi als Transportbehältnis und Medium wirken, das einen oder mehrere pharmazeutische(n) Wirkstoff(e) von einer Beeinträchtigung durch Umgebungseinflüsse sicher und zuverlässig abschirmt. Zum anderen soll das Material für die nachfolgend zu beschreibende Ausführungsform, gemäß der auf der Außenseite des Umhüllungsmaterials ein für die zu ködernde Wild-Tierart spezifisches Lockmittel aufgebracht soll, die Funktion eines Trägers übernehmen. Dabei soll das Lockmittel dauerhaft auf dem Trägermaterial haften, andererseits dieses jedoch nicht im Hinblick auf seine physikalischen Eigenschaften beeinträchtigen oder sogar durchdringen und damit die Qualität des pharmazeutischen Wirkstoffes bzw. der pharmazeutischen Wirkstoffe beeinträchtigen.

Grundsätzlich sind als Material zur Umhüllung des Wirkstoffes und zur Erfüllung der Trägerfunktion beliebige Materialien ohne Beschränkung geeignet, sofern sie die vorstehend genannten Funktionen übernehmen können.

In einer bevorzugten Ausführungsform umfaßt der erfindungsgemäße Tierköder zusätzlich zu dem bzw. den pharmazeutischen Wirkstoff(en) und dem diese(n) umhüllenden Material ein für die zu ködernde Wild-Tierart spezifisches Lockmittel, das auf der Außenseite des den/die pharmazeutischen Wirkstoff(e) vollständig umhüllenden Materials aufgebracht ist. Grundsätzlich muß es sich hierbei um ein Material handeln, das die zu ködernde Wild-Tierart aufgrund seines Aussehens, Geruchs bzw. Geschmacks anlockt, gegebenenfalls diese Tierart zum (artgerechten) Spielen mit dem Köder veranlaßt und letztendlich auch dazu veranlaßt, den Köder anzunehmen bzw. zu zerbeißen. Es hat sich gezeigt, daß die Chance der Annahme bzw. Aufnahme des Köders durch wildlebende Tiere, beispielsweise Füchse, wesentlich verbessert werden kann, wenn ein Lockmittel auf der Außenseite des Umhüllungsmaterials aufgebracht ist.

In einer besonders bevorzugten Ausführungsform handelt es sich bei einem derartigen Lockmittel um den Ernährungsgewohnheiten der spezifischen Tierart angepaßte, natürliche, von natürlichen Produkten abgeleitete oder vollsynthetische Produkte, beispielsweise Tiermehl, Fleischmehl, Fischmehl und/oder deren Hydrolysate und/oder deren Mischungen. Wenn derartige Materialien als Lockmittel verwendet werden, können insbesondere Füchse an dem erfindungsgemäßen Tierköder interessiert werden und nehmen diesen in kürzester Zeit an.

Gemäß einer weiteren bevorzugten Ausführungsform umfaßt der erfindungsgemäße Tierköder zusätzlich ein (üblicherweise als "Marker" bezeichnetes) Mittel, mit dem die Aufnahme des Köders durch ein Wildtier nach dessen Erlegen bzw. Einfangen und Töten überprüft werden kann. Derartige Mittel sind aus dem Stand der Technik als solche bekannt. Bewährt haben sich Mittel wie Tetracyclin oder damit verwandte Verbindungen, da diese auch nach längerer, für praktische Verhältnisse relevanter Zeit noch im toten Tierkörper nachgewiesen werden können.

Im Erprobungsstadium hat sich zum Nachweis der Aufnahme des Köders durch zum Test eingesetzte, in Gefangenschaft lebende Wildtiere auch Methylenblau bewährt. Beispielsweise färbt sich damit das Maul der Tiere tief blau, so daß man vom Rand des Käfigs aus erkennen kann, daß das Tier den erfindungsgemäßen, einen pharmazeutischen Wirkstoff wie z.B. Impfstoff enthaltenden Köder angenommen hat.

Gemäß einer bevorzugten Ausführungsform umfaßt das den pharmazeutischen Wirkstoff bzw. die pharmazeutischen Wirkstoffe vollständig umhüllende Material ein Material, das auf der dem/den Wirkstoff(en) zugewandten Seite mit diesem/diesen verträglich ist. Dies ist deswegen ein zwingendes Erfordernis, weil vermieden werden muß, daß das Umhüllungmaterial den pharmazeutischen Wirkstoff bzw. die Kombination pharmazeutischer Wirkstoffe hinsichtlich Aktivität und/oder Wirksamkeit selbst bei Auslage über längere Zeit beeinträchtigt.

Für die Auslegung unter Praxisbedingungen, die teilweise auch erhöhte Temperaturen (bei Sonnenbestrahlung auf dunklem Waldboden) oder Einfluß von Feuchtigkeit (Regen, Nebel, etc.) umfassen, ist es darüber hinaus ein zwingendes Erfordernis, daß das den pharmazeutischen Wirkstoff umhüllende Material auf der Außenseite feuchtigkeitsbeständig, wärmebeständig und lichtbeständig ist. Es sollte darüber hinaus eine hohe mechanische Festigkeit aufweisen, besonders bevorzugt eine Bruchkraft (bestimmt nach DIN 53112) von > 70 N/15mm in Längsrichtung bzw. > 35 N/15mm in Querrichtung.

Aufgrund von Gesichtspunkten des Umweltschutzes sollte das Material darüber hinaus ein Material sein, das im Laufe der Zeit, d.h. für den Fall, daß der erfindungsgemäße Tierköder nicht vom Wildtier angenommen wird und über längere Zeit in freier Natur liegen bleibt, im Laufe der Zeit verrottet bzw. sich zu Materialien zersetzt, die umweltverträglich sind.

In einer weiter bevorzugten Ausführungsform umfaßt der Tierköder ein den/die pharmazeutischen Wirkstoff(e) vollständig umhüllendes Material, das ein Flächengewicht im Bereich von 50 bis 150 g/m² aufweist. Ein Material mit einem über dem oberen Grenzwert liegenden Flächengewicht ist wegen der Wahrscheinlichkeit, daß die Wildtiere dieses nicht zerbeißen können, nicht geeignet. Ein Material mit einem unter dem unteren Grenzwert liegenden Flächengewicht ist demgegenüber zu wenig beständig gegen Umwelteinflüsse und weist eine zu geringe mechanische Festigkeit auf.

In einer weiter bevorzugten Ausführungsform des erfindungsgemäßen Tierköders ist das den/die pharmazeutischen Wirkstoff(e) vollständig umhüllende Material ein Verbundmaterial, das mit Vorteil ein Papier und ein synthetisches Polymer umfaßt. Besonders bevorzugt ist als Papier ein Kraftpapier mit einem Flächengewicht von 40 bis 100 g/m² und als Polyethylen ein Low-Density-Polyethylen (LDPE) mit einem Schmelzindex von etwa 4 g/10 min, einer Dichte von etwa 0,92 g/cm³ und einem Flächengewicht von 10 bis 50 g/m².

Es entspricht einer besonders bevorzugten Ausführungsform, daß der erfindungsgemäße Tierköder in Form eines gegebenenfalls mit einem für die zu ködernde Wild-Tierart spezifischen Lockmittel auf der Außenseite beaufschlagten, zwischen zwei Lagen eines Verbundmaterials einen oder mehrer pharmazeutische(n) Wirkstoff(e) enthaltenden und an den Außenrändern dicht versiegelten Wirkstoff-Päckchens vorliegt. Zu dessen Herstellung wird aus dem Umhüllungsmaterial, vorzugsweise dem Verbundmaterial, eine Art "Tasche" gebildet, die aus zwei Lagen des Umhüllungsmaterials, bevorzugt des Verbundmaterials, besteht. In diese Tasche wird der pharmazeutische Wirkstoff oder die Kombination mehrerer pharmazeutischer Wirkstoffe eingefüllt. Dies geschieht unter den nachfolgend noch im einzelnen beschriebenen Bedingungen. Nach Einfüllen einer definierten Menge des Wirkstoffs, die im Bereich von 0,1 bis 10 ml, besonders bevorzugt von 1 bis 3 ml, liegen kann, wird das Umhüllungsmaterial bzw. Verbundmaterial an den Außenrändern der "Tasche" dicht versiegelt und so ein den pharmazeutischen Wirkstoff bzw. die Kombination pharmazeutischer Wirkstoffe enthaltendes Wirkstoff-Päckchen bereitgestellt.

Grundsätzlich ist die Form des erfindungsgemäßen Tierköders nicht in irgendeiner Weise beschränkt. So kann der in Form eines Wirkstoff-Päckchens vorliegende erfindungsgemäße Tierköder eine rechteckige, dreieckige, runde oder ovale Form aufweisen. Bevorzugt wird eine rechteckige, besonders bevorzugt eine quadratische Form. Dies hängt nicht nur mit der nachfolgend im einzelnen zu beschreibenden Herstellungsweise des erfindungsgemäßen Tierköders zusammen, sondern auch mit seinen Eigenschaften im praktischen Gebrauch, beispielsweise bei der Ausbringung in freier Natur. So wird in jüngerer Zeit ein Köderkörper verlangt, der sich von seinen äußeren Abmessungen her dazu eignet, von einem Flugzeug aus abgeworfen zu werden. Die bisherigen, häufig in Form von zylindrischen Scheiben vorliegenden Köder waren dafür nicht optimal geeignet. Die erfindungsgemäß bevorzugten Tierköder in Form eines rechteckigen bzw. quadratischen Wirkstoff-Päckchens weisen jedoch den Vorteil auf, daß sie bei Abwurf aus einem Flugzeug keinen Fallkörper darstellen, sondern aufgrund ihres einer Linse angenäherten Profils ein Flugkörper sind. Diese segeln in einer enge Spiralen beschreibenden Flugbahn in relativ spitzem Winkel zur Erde. Dadurch wird vermieden, daß sie - beispielsweise bei unbeabsichtigtem Aufprallen auf Gegenstände (Häuser, etc.) oder Personen bzw. Tiere - Beschädigungen bzw. Verletzungen verursachen. Darüber hinaus kann aufgrund der definierten Flugeigenschaften bei definierter Form der Wirkstoff-Päckchen auch der Ort der Landung mit relativ großer Sicherheit abgeschätzt werden, so daß sich eine gute Reproduzierbarkeit bzw. Protokollierbarkeit der Auslegung ergibt.

In Abhängigkeit von der Füllung mit pharmazeutischem Wirkstoff kann die Dicke des erfindungsgemäßen Tierköders unterschiedlich stark sein, ohne daß es auf diese kritisch ankommt. In einer besonders bevorzugten Ausführungsform weist das Wirkstoff-Päckchen des erfindungsgemäßen Tierköders eine Dicke im Bereich von 1 bis 3 mm auf.

Der Tierköder gemäß der vorliegenden Erfindung kann in einem industriell adaptierbaren Verfahren in großen Mengen preiswert hergestellt werden und weist alle vorteilhaften Eigenschaften eines im freien auslegbaren Tierköders auf. So ist er bei üblichen Auslegungstemperaturen und -feuchten ohne Stabilitäts- und Wirkungsverlust lagerbar und kann ohne Schwierigkeiten gehandhabt, insbesondere für die manuelle oder maschinelle Auslegung vorbereitet werden. Er eignet sich sowohl für die manuelle Auslegung am Boden als auch für die aus ökonomischen Gründen bevorzugte Auswerfung vom Flugzeug aus. Aufgrund der Trennung der Herstellung des den pharmazeutischen Wirkstoff bzw. mehrere pharmazeutische Wirkstoffe umfassenden Wirkstoff-Päckchens von der möglichen Aufbringung eines Lockmittels wird die Lagerung erleichtert. Dabei macht sich bei dem erfindungsgemäßen Tierköder besonders vorteilhaft bemerkbar, daß sein geringes Volumen eine Lagerung auf engem (Kühl-) Raum ermöglicht. Die auf den Zeitpunkt spätestens kurz vor der Ausbringung verlegte Aufbringung des Lockmittels macht das separate Verpacken einzelner Köder-Formkörper zur Vermeidung eines Verklebens oder zur Verhinderung des Kontakts mit dem Menschen oder zur Vermeidung des Austretens der für den Menschen unangenehmen Gerüche des Lockmittels überflüssig.

Letzlich ist auch die Auslegung in automatisierter Form besonders erleichtert, da es die erfindungsgemäßen, in Form eines lockmittelfreien Wirkstoff-Päckchens vorliegenden Tierköders ermöglichen, die einzelnen Wirkstoff-Päckchen vollautomatisch im Flugzeug mit Lockmittel zu beaufschlagen und anschließend nach genau festgelegtem Flugprogramm durch Abwerfen auszulegen. Die Gefahr von Schäden durch auf dem Boden auftreffende Tierköder-Wirkstoff-Päckchen besteht bei den erfindungsgemäßen Ködern im Gegensatz zum Stand der Technik nicht mehr.

Die erfindungsgemäßen Tierköder werden darüber hinaus von den Wildtieren, beispielsweise von Füchsen, optimal angenommen. Die spezielle Form des Köders verhindert das (artgerechte) Spielen der Wildtiere, beispielsweise der Füchse, mit dem Köder nicht. Außerdem führt das Einbeißen der Tiere in den als Wirkstoff-Päckchen vorliegenden Köder zu einem optimalen Kontakt des pharmazeutischen Wirkstoffs bzw. der Kombination pharmazeutischer Wirkstoffe mit den oralen Schleimhäuten des Tieres. Eine angestrebte Behandlung von Krankheiten wildlebender Tiere oder Vorbeugung gegen Krankheiten bei wildlebenden Tieren ist damit mit den erfindungsgemäßen Ködern in zuverlässiger und nachprüfbarer Weise möglich.

Nachfolgend wird das Verfahren zur Herstellung des erfindungsgemäßen Tierköders näher erläutert. Dieses umfaßt als ersten Schritt, daß man ein zum vollständigen Umhüllen des/der pharmazeutischen Wirkstoffe(s) geeignetes Material auswählt und in für die weiteren Produktionsschritte geeigneter Weise vorlegt. Dieses Material sollte einerseits so flexibel sein, daß es unter den nachfolgend genauer angegebenen Bedingungen bzw. in den folgenden Schritten bearbeitet bzw. gehandhabt werden kann, andererseits so stabil bzw. fest sein, daß es den Anforderungen an Haltbarkeit bzw. Stabilität genügt, um eine Schutzfunktion für den/die pharmazeutischen Wirkstoff(e) übernehmen zu können.

Vorteilhafterweise wird das Material zu einer Tasche bzw. einem mindestens dreiseitig eingefaßten Beutel geformt, um später den pharmazeutischen Wirkstoff bzw. eine Kombination pharmazeutischer Wirkstoffe aufnehmen zu können. In bevorzugter Weise geschieht dies so, daß zwei Lagen des ausgewählten Materials, beispielsweise des oben beschriebenen Verbundmaterials, so angeordnet werden, daß dessen eine Seite, die mit dem/den pharmazeutischen Wirkstoff(en) verträglich ist, bei beiden Lagen nach innen zu liegen kommt, während dessen andere Seite, die besondere Eigenschaften hinsichtlich Feuchtigkeitsbeständigkeit, Wärmebeständigkeit und Lichtbeständigkeit und eine hohe mechanische Festigkeit aufweist, nach außen zu liegen kommt. Besonders bevorzugt kann die Herstellung der Tasche in einer Weise erfolgen, die zu einem auf Rollen o. ä. lieferbaren Material für die spätere automatisierte Abpackung des/der pharmazeutischen Wirkstoffe(s) führt.

In einer weiter bevorzugten Ausführungsform des Verfahrens wird zur Umhüllung des/der pharmazeutischen Wirkstoffe(s) ein Material verwendet, das ein Flächengewicht im Bereich von 50 bis 150 g/m² aufweist. Mit einem derartigen Material wird in vorteilhafter Weise einerseits die notwendige Festigkeit und Stabilität des Umhüllungsmaterials und die Erfüllung der Schutzfunktion für den bzw. die pharmazeutischen Wirkstoff(e) erreicht, andererseits jedoch auch ein geringes Gewicht und damit eine bessere Handhabbarkeit bei der praktischen Verwendung.

Mit besonderem Vorteil läßt sich das vorstehend bereits beschriebene Verbundmaterial in dem erfindungsgemäßen Verfahren verwenden, das ein Papier und ein synthetisches Polymer umfaßt. Dabei zeigt bei der Verarbeitung dieses Verbundmaterials das Polymer, beispielsweise ein Low-Density-Polyethylen (LDPE) mit einem Schmelzindex von etwa 4 g/10 min, einer Dichte von etwa 0,92 g/cm³ und einem Flächengewicht von 10 bis 50 g/m², zum pharmazeutischen Wirkstoff, während das Papier, beispielsweise ein Kraftpapier, besonders bevorzugt mit einem Flächengewicht von 40 bis 100 g/m², nach außen zeigt. Dadurch wird sichergestellt, daß der/ die pharmazeutische(n) Wirkstoff(e) von dem Verbundmaterial in ihrer Aktivität und Wirksamkeit nicht unkontrolliert beeinflußt werden. Andererseits wird mit der nach außen zeigenden Papierschicht sichergestellt, daß eine optimale Beständigkeit gegen Umgebungsbedingungen (Feuchtigkeit, Wärme, Licht) gegeben ist und darüber hinaus die Funktion erfüllt werden kann, gegebenenfalls ein Träger für das für die zu ködernde Wild-Tierart spezifische Lockmittel zu sein.

Erfindungsgemäß wird im nächsten Verfahrensschritt die aus dem Umhüllungsmaterial gebildete "Tasche" mit dem pharmazeutischen Wirkstoff bzw. einer Kombination mehrerer pharmazeutischer Wirkstoffe gefüllt. Diese(r) kann/können in Reinsubstanz vorliegen oder können eine Mischung, Lösung, Suspension o. ä. miteinander oder mit Träger- oder Hilfsstoffen sein. Es können dazu beliebige, an sich aus dem Stand der Technik bekannte Träger- und Hilfsstoffe verwendet werden.

Wie bereits oben angegeben, kann zusammen mit dem/den pharmazeutischen Wirkstoff(en) auch ein Marker für den oben angegebenen Zweck mit eingefüllt werden. Dieser muß natürlich mit dem/den pharmazeutischen Wirkstoff(en) genauso verträglich sein wie mit dem Umhüllungsmaterial.

Das Abfüllen erfolgt jedenfalls unter Bedingungen, die für den/die pharmazeutischen Wirkstoff(e) nicht in irgendeiner Weise schädlich sind. Besonders dürfen unter den Bedingungen des Abfüllens des/der pharmazeutischen Wirkstoffe(s) dessen/deren Aktivität oder Wirksamkeit nicht leiden. Hierzu kann beispielsweise ein Abfüllen unter Schutzbegasung unter Einhaltung steriler Bedingungen und/oder auch ein Abfüllen unter Kühlung gehören. Die speziellen Bedingungen richten sich nach dem jeweiligen pharmazeutischen Wirkstoff und sind dem Fachmann als solches ebenfalls bekannt.

Erfindungsgemäß besonders bevorzugt ist für das vorliegende Verfahren die Abfüllung eines flüssigen Wirkstoffs, beispielsweise die Abfüllung eines flüssigen Impfstoffs wie eines flüssigen Oral-Impfstoffs. Mit besonderem Vorteil wird ein flüssiger Oral-Impfstoff gegen Tollwut bei Wild-Tieren wie beispielsweise bei Füchsen abgefüllt. Die Menge liegt dabei bevorzugt bei 0,5 bis 5 ml, besonders bevorzugt bei 1 bis 3 ml.

In einer weiteren, bevorzugten Ausführungsform wird - wie oben beschrieben - dem Impfstoff ein Marker zugesetzt, der eine Kontrolle der Annahme des Köders bzw. Aufnahme des Impfstoffs nach Erlegen bzw. Fangen und Töten des Wildtiers ermöglicht. Je nach der Aktivität des Markers werden mehr oder weniger große Mengen eingesetzt. Deren Menge liegt im Bereich des Könnens eines Fachmanns auf dem Gebiet der Köderherstellung.

Im nächsten Schritt wird der pharmazeutische Wirkstoff bzw. die Kombination aus mehreren pharmazeutischen Wirkstoffen und gegebenenfalls einem Marker vollständig mit dem vorstehend genannten Umhüllungsmaterial umhüllt. Dies geschieht in bevorzugter Weise dadurch, daß man die befüllte "Tasche" vollständig verschließt bzw. versiegelt. Dies kann beispielsweise geschehen durch Heißversiegeln der Kanten in an sich bekannter Weise. Durch punktuelles, d.h. nur an den Kanten der "Tasche" erfolgendes Versiegeln wird ein schädlicher Einfluß der Versiegelungswärme auf den pharmazeutischen Wirkstoff vermieden. Dadurch wird erfindungsgemäß sichergestellt, daß die Aktivität bzw. Wirksamkeit des/der pharmazeutischen Wirkstoffe(s) durch diesen Schritt nicht beeinträchtigt wird. Auf diesem Weg wird also ein den/die pharmazeutischen Wirkstoff(e) enthaltendes Wirkstoff-Päckchen hergestellt, in dem der/die Wirkstoff(e) geschützt vor Beeinträchtigungen durch die Umgebung vorliegt/vorliegen und eine Wechselwirkung mit dem möglicherweise später aufzutragenden Lockmittel nicht zu befürchten ist.

Das Wirkstoff(e) enthaltende Päckchen kann dann unter geeigneten Bedingungen gelagert werden, bis es weiterverarbeitet oder ausgebracht wird. Mit dem Tierköder gemäß der Erfindung ist es insbesondere möglich, bei Herstellung von Tollwutvirus-Impfstoffpäckchen, die nur zu bestimmten Zeiten im Jahr (März/April und September/Oktober) ausgelegt werden, für die "Kampagne" auf Vorrat zu produzieren und die fertigen Wirkstoff-Päckchen ohne Titerverlust des Impfstoffs unter Kühlung zu lagern.

Die Lagerung erfolgt bei Bedingungen, die die Aktivität und Wirksamkeit des/der pharmazeutischen Wirkstoffe(s) nicht beeinträchtigen und damit weitgehend von dessen Natur abhängen, mit Vorteil bei Dunkelheit und/oder unter Kühlung, bevorzugt bei Temperaturen unter 10 °C, besonders bevorzugt bei Temperaturen im Bereich von 0 bis 5 °C.

Zum gegebenen Zeitpunkt kann dann der erfindungsgemäße Tierköder zur Vorbeugung gegen und/oder zur Bekämpfung von Krankheiten wildlebender Tiere ausgebracht bzw. ausgelegt werden. In einer bevorzugten Ausführungsform geschieht dies, ohne daß vorher ein für die zu ködernde Tierart spezifisches Lockmittel auf die Außenseite des Wirkstoffpäckchens aufgebracht wurde.

Es entspricht jedoch einer besonders bevorzugten Ausführungsform des erfindungsgemäßen Verfahrens, daß vor der Ausbringung des Köders auf dessen Außenseite, d. h. auf die Außenseite des den/die pharmazeutischen Wirkstoff(e) umgebenden Umhüllungsmaterials, bevorzugt die Papierseite des Umhüllungsmaterials, ein Lockmittel aufgebracht wird, das für die zu ködernde Tierart spezifisch ist. Bei der bevorzugten Verwendung des erfindungsgemäßen Köders für Füchse wird - wie oben beschrieben - beispielsweise Tiermehl, Fleischmehl, Fischmehl und/oder eines von deren Hydrolysaten oder eine Mischung aus den genannten Materialien auf die Außenseite des Wirkstoff-Päckchens aufgebracht. Dies geschieht in einer weiter bevorzugten Ausführungsform durch Aufstreichen, Aufbürsten oder Aufsprühen eines flüssigen, härtbaren Lockmittels auf das Wirkstoffpäckchen oder Eintauchen des Wirkstoffpäckchens in ein flüssiges, härtbares Lockmittel. Zur Durchführung wird das Lockmittel je nach der Natur von dessen Inhaltsstoffen durch Wärmeeinwirkung aufgeschmolzen oder in einem geeigneten Hilfsmittel gelöst oder aufgeschlämmt und dann in der genannten Weise auf die Außenseite des Wirkstoffpäckchens aufgebracht.

Die Menge an Lockmittel ist nicht kritisch. Aus ökonomischen Gründen wird jedoch die Lockmittel-Menge möglichst gering gehalten und nicht höher gewählt, als erforderlich ist, um einen Effekt des Anlockens des Wild-Tieres zu bewirken. Beispielshaft wird eine Gesamt-Lockmittel-Menge von etwa 1 bis 5 g auf das Wirkstoff-Päckchen beidseitig aufgebracht, bevorzugt 1 bis 2 g. Je nach Lockmittel-Menge ergibt sich somit eine Dicke des erfindungsgemäßen Köders im Bereich von 1 bis 5 mm.

Die Ausbringung des so nach dem erfindungsgemäßen Verfahren hergestellten Tierköders erfolgt dann in an sich bekannter Weise, zum Beispiel durch manuelles Auslegen oder Abwerfen von Flugzeugen oder Hubschraubern. Letzteres ist wegen der Möglichkeit der Automatisierung (und des Fehlens des Kontakts des Menschen zum Einzelköder beim Auslegen) und natürlich auch aus ökonomischen Gründen bevorzugt. Man hat heute zudem die Möglichkeit, die Ausbringung der Köder per Flugzeug reproduzierbar zu gestalten und damit genau zu überwachen, welche Fläche mit einer bestimmten Menge an Einzelködern versorgt wurde, um einen Erfolg der Behandlungs- oder Vorbeuge-Aktion nachvollziehen zu können.

In diesem Sinn entspricht es einer besonders bevorzugten Ausführungsform des erfindungegemäßen Verfahrens, daß man die Köder nach der Lagerung (oder gegebenenfalls auch unmittelbar nach dem Befüllen der Wirkstoff-Päckchen mit dem/den pharmazeutischen Wirkstoff(en)) auf einer in einem Flugzeug installierten Vorrichtung installiert und in Abstimmung mit den Flugdaten gegebenenfalls mit dem entsprechend konditionierten, beispielsweise geschmolzenen oder suspendierten, Lockmittel beaufschlagt und dann sofort durch automatisierten Abwurf ausbringt. Der Vorteil dieser Vorgehensweise besteht darin, daß man die Tierköder gemäß der Erfindung unter optimalen Bedingungen herstellen und unter geringstmöglichem Raumbedarf lagern kann und sie dann beispielsweise unter Kühlung transportieren und gegebenenfalls nach Aufbringung des Lockmittels automatisiert ausbringen kann. Diese Vorgehensweise vermeidet Kontakt des Menschen mit Einzelködern, die ein Wildtier nachträglich wahrnimmt und es davon abhält, den Köder anzunehmen. Darüber hinaus wird mit diesem Vorgehen eine Verminderung der Qualität des Köders weitgehend vermieden. Beispielsweise trägt die optimale Lagerung und der optimale Transport des Wirkstoff-Päckchens dazu bei, den natürlichen Aktivitätsverlust und Wirksamkeitsverlust des/der pharmazeutischen Wirkstoffe(s) sehr gering zu halten, wenn nicht sogar zu vermeiden. Der Lockstoff kann "frisch" aufgebracht werden und damit die anlockende Funktion optimal entfalten. Darüber hinaus kann bei Auftragung des warmen (z. B. geschmolzenen) Lockstoffs auf das Wirkstoffpäckchen unmittelbar vor dem Abwurf aus dem Flugzeug der Luftzug die Kühlung übernehmen, so daß bei Auftreffen auf dem Boden der Lockstoff gehärtet und trotzdem frisch ist.

Erfindungsgemäß wird der Tierköder gemäß der Erfindung, wie er vorstehend beschrieben wurde, dazu verwendet, bei wildlebenden Tieren Krankheiten zu behandeln oder dem Auftreten von Krankheiten vorzubeugen. In einer besonders bevorzugten Verwendung werden beispielsweise Füchse mit einem gegen den Tollwut-Virus gerichteten Impfstoff vorbeugend gegen Wildtollwut geimpft. Die Füchse nehmen den nach dem vorstehend beschriebenen Verfahren hergestellten und flächendeckend ausgebrachten Tierköder bereitwillig an. Die Immunisierung der Füchse, die den Köder angenommen haben, ist nachhaltig und führt zu einem signifikanten Rückgang der Fälle von Wildtollwut in dem Ausbringgebiet.

Die Erfindung wird durch das nachfolgende Beispiel näher erläutert.

### Beispiel

Ein Verbundmaterial aus einem Kraftpapier mit einem Flächengewicht von 60 g/m² und einem Low-Density-Polyethylen (LDPE) mit einem Schmelzindex von etwa 4 g/10 min, einer Dichte von 0,923 g/cm³ und einem Flächengewicht von 30 g/m² (Hersteller: Firma Maria Soel GmbH, Nidda) in Folienform wurde auf einer üblichen Flachbeutelmaschine (4-Seiten-Siegelrandbeutelmaschine) zur Befüllung und zum Verschließen heißsiegelfähiger Verbundfolien in Rollenform vorgelegt. Das Material wurde unter üblichen Bedingungen für die Abfüllung von im Veterinärbereich verwendeten Impfstoffen bei einer Temperatur unter 10 °C mit 1 bis 2 ml eines üblichen flüssigen, oral wirksamen Impfstoffs gegen Wildtollwut, dem Tetracyclin als Marker in üblicher Menge zugesetzt war, befüllt. Die so befüllten Beutel wurden anschließend in der Vorrichtung heißversiegelt.

In einem Durchlauf wurden die so heißversiegelten Beutel auf Endlosrollen aufgerollt und in Rollenform gelagert. Bei einem anderen Durchlauf wurden die Beutel unmittelbar nach dem Heißversiegeln auf Auswurfgröße geschnitten und in Form einer Ansammlung von Einzelbeuteln gelagert.

Der Titer des Impfstoffs wurde unmittelbar nach dem Heißversiegeln bestimmt. Er war (im Rahmen der Meßgenauigkeit) gegenüber dem ursprünglich vor dem Abfüllen eingesetzten Impfstoff identisch.

Die befüllten Wirkstoff-Päckchen wurden bei Temperaturen zwischen 1 und 4 °C drei Monate im Dunkeln gelagert. Nach Ablauf dieser Zeit des Lagerns unter den angegebenen Bedingungen war der Titer nur geringfügig abgefallen.

Die vorher in Form von einzelnen Beuteln hergestellten Wirkstoff-Päckchen wurden durch Aufstreichen einer geschmolzenen Mischung aus Fischmehl und Tiermehl bestrichen und einer Kühlstrecke zugeführt. Nach Abkühlen ergaben sich einzelne, nicht aneinander klebende, mit der Lockstoff-Mischung beaufschlagte Wirkstoff-Beutel, die sich "in bulk" lagern und durch Abwerfen vom Flugzeug auslegen ließen.

Die vorher in Rollenform gelagerten Wirkstoff-Päckchen wurden in einer Kühlbox in einem für den Abwurf von Ködern geeigneten Flugzeug eingebaut und nach einem eingestellten, nach den Flugbedingungen und der Abwurfdichte gesteuerten Rhythmus von der Rolle abgerollt, durch Aufstreichen mit einer Lockmittel-Mischung aus geschmolzenem Fischmehl und Tiermehl beaufschlagt, vom mit Lockmittel beaufschlagten Band abgeschnitten und abgeworfen. Das Lockmittel erhärtete im Flugwind.

Die ausgeworfenen Tierköder gemäß der Erfindung wurden bereitwillig von Füchsen angenommen und auch zerbissen. Es konnte somit ein flächendeckender Impferfolg erzielt werden, wie anhand von erlegten Füchsen, in denen der Marker nachgewiesen werden konnte, nachgeprüft wurde.

Es konnte somit ein für die Impfung wirksamer, industriell preiswert und einfach herstellbarer, einfach handhabbarer Tierköder hergestellt werden, dessen Impfstoff nicht durch das Herstellungsverfahren bereits eine Verschlechterung erfahren hatte.

## Patentansprüche

1. Tierköder zur Vorbeugung gegen und/oder Behandlung von Krankheiten wildlebender Tiere mit einem oder mehreren pharmazeutischen Wirkstoff(en), bestehend aus einem den/die pharmazeutischen Wirkstoff(e) vollständig umhüllenden, mit diesem/diesen nicht in Wechselwirkung tretenden Material, das gleichzeitig so beschaffen ist, daß ein gegebenenfalls auf der Außenseite der daraus gebildeten vollständigen Umhüllung aufgebrachtes, für die zu ködernde Tierart spezifisches Lockmittel darauf haftet, wobei das Lockmittel kein Fleischköder ist.

2. Tierköder nach Anspruch 1, umfassend zusätzlich ein für die zu ködernde Wild-Tierart spezifisches Lockmittel auf der Außenseite des den/die pharmazeutischen Wirkstoff(e) vollständig umhüllenden Materials.

3. Tierköder nach Anspruch 1 oder 2, umfassend zusätzlich ein Mittel (Marker), mit dem die Aufnahme des Köders durch ein Wild-Tier nach dessen Erlegen bzw. Einfangen und Töten überprüft werden kann, bevorzugt Tetracyclin oder Methylenblau.

4. Tierköder nach einem oder mehreren der Ansprüche 1 bis 3, umfassend als pharmazeutischen Wirkstoff einen Impfstoff, bevorzugt einen flüssigen Impfstoff, weiter bevorzugt einen für die orale Aufnahme durch Wildtiere geeigneten flüssigen Impfstoff, noch mehr bevorzugt einen Impfstoff gegen Tollwut.

5. Tierköder nach einem oder mehreren der Ansprüche 1 bis 4, umfassend als den/die pharmazeutischen Wirkstoff(e) vollständig umhüllendes Material ein Material, das auf der dem/den Wirkstoff(en) zugewandten Seite mit diesem/diesen verträglich ist und auf der Außenseite feuchtigkeitsbeständig, wärmebeständig und lichtbeständig ist und eine hohe mechanische Festigkeit aufweist.

6. Tierköder nach Anspruch 5, worin das den/die pharmazeutischen Wirkstoff(e) vollständig umhüllende Material ein Flächengewicht im Bereich von 50 bis 150 g/m² aufweist.

7. Tierköder nach einem oder mehreren der Ansprüche 1 bis 6, worin das den/die pharmazeutischen Wirkstoff(e) vollständig umhüllende Material ein Papier und ein synthetisches Polymer umfassendes Verbundmaterial ist, besonders bevorzugt ein Verbundmaterial aus Kraftpapier und Low-Density-Polyethylen mit einem Schmelzindex von etwa 4 g/10 min und einer Dichte von etwa 0,92 g/cm³.

8. Tierköder nach einem oder mehreren der Ansprüche 1 bis 7, welcher in Form eines gegebenenfalls mit einem für die zu ködernde Wild-Tierart spezifischen Lockmittel auf der Außenseite beaufschlagten, zwischen zwei Lagen eines Verbundmaterials einen oder mehrere pharmazeutische(n) Wirkstoff(e) enthaltenden und an den Außenrändern dicht versiegelten Wirkstoff-Päckchens vorliegt.

9. Tierköder nach Anspruch 8, welcher eine rechteckige, bevorzugt quadratische, dreieckige, runde oder ovale Form aufweist.

10. Tierköder nach einem oder mehreren der Ansprüche 1 bis 9, welcher eine Dicke im Bereich von 1 bis 5 mm aufweist.

11. Tierköder nach einem oder mehreren der Ansprüche 1 bis 10, worin das für die zu ködernde Wild-Tierart spezifische Lockmittel Tiermehl, Fleischmehl, Fischmehl und/oder deren Hydrolysate oder deren Mischungen umfaßt.

12. Verfahren zur Bereitstellung eines einen oder mehrere pharmazeutische(n) Wirkstoff(e) enthaltenden Tierköders zur Vorbeugung gegen und/oder Behandlung von Krankheiten wildlebender Tiere, gekennzeichnet durch die Schritte, daß man
(a) ein zum vollständigen Umhüllen des/der pharmazeutischen Wirkstoffe(s) geeignetes und mit diesem nicht in Wechselwirkung tretendes Material, das gleichzeitig so beschaffen ist, daß ein gegebenenfalls auf der Außenseite der daraus gebildeten vollständigen Umhüllung aufgebrachtes für die zu ködernde Tierart spezifisches Lockmittel darauf haftet, vorlegt und zu einer Tasche formt, wobei das Lockmittel kein Fleischköder ist;
(b) die Tasche unter für den/die pharmazeutischen Wirkstoff(e) unschädlichen Bedingungen mit dem/den pharmazeutischen Wirkstoff(en) befüllt und unter Bildung einer vollständigen Umhüllung versiegelt;
(c) das so gebildete Wirkstoffpäckchen gegebenenfalls unter geeigneten Bedingungen lagert; und
(d) ausbringt.

13. Verfahren nach Anspruch 12, worin die Ausbringung des Köders in einem Abwerfen aus dem Flugzeug besteht.

14. Verfahren nach Anspruch 12 oder 13, gekennzeichnet durch den zusätzlichen Schritt, daß man das Wirkstoffpäckchen
(a) nach Schritt b und vor Schritt c; oder
(b) nach Schritt c und vor dem Ausbringungsschritt d, bevorzugt unmittelbar vor der Ausbringung, besonders bevorzugt unmittelbar vor dem Abwerfen aus dem Flugzeug
mit einem für die zu ködernde Tierart spezifischen Lockmittel beaufschlagt.

15. Verfahren nach Anspruch 14, worin die Beaufschlagung des Lockmittels durch Aufstreichen, Aufbürsten oder Aufsprühen eines flüssigen, härtbaren Lockmittels auf das Wirkstoffpäckchen oder Eintauchen des Wirkstoffpäckchens in ein flüssiges, härtbares Lockmittel erfolgt.

16. Verfahren nach einem oder mehreren der Ansprüche 12 bis 15, worin als Material zur Umhüllung des/der pharmazeutischen Wirkstoffe(s) ein Material verwendet wird, das auf der dem/den Wirkstoff(en) zugewandten Seite mit diesem/diesen verträglich ist und auf der Außenseite feuchtigkeitsbeständig, wärmebeständig und lichtbeständig ist und eine hohe mechanische Festigkeit aufweist.

17. Verfahren nach einem oder mehreren der Ansprüche 12 bis 16, worin als Material zur Umhüllung des/der pharmazeutischen Wirkstoffe(s) ein Material verwendet wird, das ein Flächengewicht im Bereich von 50 bis 150 g/m² aufweist.

18. Verfahren nach einem oder mehreren der Ansprüche 12 bis 17, worin als den/die pharmazeutischen Wirkstoff(e) vollständig umhüllendes Material ein Papier und ein synthetisches Polymer umfassendes Verbundmaterial verwendet wird, besonders bevorzugt ein Verbundmaterial aus Kraftpapier und Low Density-Polyethylen mit einem Schmelzindex von etwa 4 g/10 min und einer Dichte von etwa 0,92 g/cm³.

19. Verfahren nach einem oder mehreren der Ansprüche 12 bis 18, worin das Abfüllen des/der pharmazeutischen Wirkstoffe(s) und das Ausbilden einer vollständigen Umhüllung unter Bedingungen erfolgen, die die Aktivität und/oder Wirksamkeit des/der pharmazeutischen Wirkstoffe(s) nicht beeinträchtigen, bevorzugt unter Sterilbedingungen und/oder unter Kühlung.

20. Verfahren nach einem oder mehreren der Ansprüche 12 bis 19, worin als pharmazeutischer Wirkstoff ein flüssiger Impfstoff, bevorzugt ein durch Wild-Tiere oral aufnehmbarer Impfstoff, besonders bevorzugt ein flüssiger, durch Wild-Tiere oral aufnehmbarer Tollwut-Impfstoff, verwendet wird.

21. Verfahren nach einem oder mehreren der Ansprüche 12 bis 20, worin die vollständige Umhüllung des/der pharmazeutischen Wirkstoffe(s) mit dem flexiblen Material durch punktuelles Heißversiegeln erfolgt.

22. Verfahren nach einem oder mehreren der Ansprüche 12 bis 21, worin die Lagerung unter Erhaltung der Aktivität und/oder Wirksamkeit des/der pharmazeutischen Wirkstoffe(s) erfolgt, bevorzugt in der Dunkelheit und unter Kühlung, bevorzugt bei Temperaturen unter 10 °C, besonders bevorzugt bei Temperaturen im Bereich von 0 bis 5 C°.

23. Tierköder nach einem oder mehreren der Ansprüche 1 bis 11 zur Verwendung bei der Vorbeugung gegen und/oder Behandlung von Krankheiten wildlebender Tiere.

24. Tierköder nach Anspruch 23 zur Verwendung bei der Bekämpfung von Wildtollwut bei Füchsen.

## Claims

1. Bait for animals for preventing and/or treating diseases of wildlife animals comprising one or more than one pharmaceutically effective substance(s), said bait consisting of a material completely enveloping said pharmaceutically effective substance(s) and not interacting therewith, said material coincidentally being of such a quality that an attracting agent specific for the animal species to be attracted and optionally applied to the outer side of said complete envelope made of said material, adheres thereto, whereby the attracting agent is not a meat bait.

2. Bait for animals according to claim 1, additionally comprising an attracting agent specific for the wildlife animal species to be attracted on the outer side of the material completely enveloping the pharmaceutically effective substance(s).

3. Bait for animals according to claim 1 or claim 2, additionally comprising an agent (marker) by which the intake of said bait by said wildlife animals after its killing or catching and killing respectively, may be examined, preferably comprising tetracycline or methylene blue.

4. Bait for animals according to one or more than one of the claims 1 to 3, comprising, as the pharmaceutically effective substance, a vaccine, preferably a liquid vaccine, more preferably a liquid vaccine suitable for oral intake by wildlife animals, even more preferred a vaccine against rabies.

5. The bait for animals according to one or more than one of the claims 1 to 4, comprising, as the material completely enveloping the pharmaceutically effective substance(s), a material compatible with the effective substance(s) on the side facing to said substance(s) and being resistant to humidity, resistant to heat and resistant to light on the outer side and having a high mechanical strength.

6. The bait for animals according to claim 5, wherein the material completely enveloping the pharmaceutically effective substance(s) has an area weight in the range of from 50 to 150 g/m².

7. The bait for animals according to one or more than one of the claims 1 to 6 wherein the material completely enveloping the pharmaceutically effective substance(s) is a composite material comprising a paper and a synthetic polymer, particularly preferred a composite material made of Kraft paper and low density polyethylene having a melt index of about 4 g/10 min and a density of about 0.92 g/cm³.

8. The bait for animals according to one or more than one of claims 1 to 7, which is in the form of a package of the effective substance(s) optionally having applied to its outer side an attracting agent specific for the wildlife animals species to be attracted and containing one or more than one pharmaceutically effective substance(s) between two layers of said composite material and being tightly sealed at the outer edges.

9. The bait for animals according to claim 8 having a rectangular, preferably square, triangular, round or oval shape.

10. Bait for animals according to one or more than one of the claims 1 to 9, having a thickness in the range of from 1 to 5 mm.

11. The bait for animals according to one or more than one of the claims I to 10. wherein the attracting agent specific for the wildlife animals species to be attracted comprises animal powder, meat powder, fish powder and/or their hydrolysates or their mixtures.

12. The process for providing a bait for animals for preventing and/or treating diseases of wildlife animals, said bait containing one or more than one pharmaceutically effective substance(s), said process being characterized by the steps of
(a) providing a material suitable for completely enveloping the pharmaceutically effective substance(s) and not interacting therewith, which concurrently is of a quality that an attracting agent applied to the outer side of the complete envelope made thereof and specific for the animal species to be attracted adheres thereto, and forming said material to an envelop, wherein the attracting agent is not a meat bait;
(b) filling said envelope, under conditions not detrimental to said pharmaceutically effective substance(s) with said pharmaceutically effective substance(s) and sealing said envelope with forming a completely sealed envelope;
(c) optionally storing the package of effective substance(s) thus formed under suitable conditions; and
(d) bringing out said package.

13. The process according to claim 12, wherein the step of bringing out said bait is a step of releasing said bait from an airplane.

14. The process according to claim 12 or claim 13, characterized by the additional step of applying to said package of effective substance(s) an attracting agent specific for the animal species to be attracted
(a) after step (b) and before (c); or
(b) after step (c) and before the step of bringing out said package (d), preferably immediately before the step of bringing out said package, particularly preferred immediately before releasing the package from the airplane.

15. The process according to claim 14, wherein the step of applying the attracting agent is a step of spreading, brushing or spraying of a liquid, hardenable attracting agent onto said package of effective substance(s) or a step of dipping said package of effective substance(s) into a liquid, hardenable attracting agent.

16. The process according to one or more than one of the claims 12 to 15, wherein, as the material for enveloping said pharmaceutically effective substance(s), a material is used which is compatible with said pharmaceutically effective substance(s) on the side facing to said substance(s) and which is resistant to humidity, resistant to heat and resistant to light on the outer side and has a high mechanical strength.

17. The process according to one or more than one of the claims 12 to 16, wherein, as the material for enveloping the pharmaceutically effective substance(s), a material is used which has an area weight in the range of from 50 to 150 g/m².

18. The process according to one or more than of the claims 12 to 17, wherein, as the material completely enveloping the pharmaceutically effective substance(s), there is used a composite material comprising a paper and a synthetic polymer, particularly preferably a composite material made of Kraft paper and low density polyethylene having a melt index of about 4 g/10 min and a density of about 0.92 g/cm³.

19. The process according to one or more than one of claims 12 to 18, wherein the steps of filling in the pharmaceutically effective substance(s) and of forming the complete envelope are conducted under conditions not detrimental to the activity and/or effectivity of pharmaceutically effective substance(s), preferably under sterile conditions and/or under cooling.

20. The process according to one or more than one of the claims 12 to 19, wherein, as the pharmaceutically effective substance, there is used a liquid vaccine, preferably a vaccine suitable for oral intake by wildlife animals, particularly preferable a liquid rabies vaccine for oral intake by wildlife animals.

21. The process according to one or more than one of claims 12 to 20, wherein the steps of completely enveloping said pharmaceutically effective substance(s) with the flexible material is conducted by spot heat sealing.

22. The process according to one or more than one of claims 12 to 21, wherein the step of storing is conducted by maintaining the activity and/or effectiveness of said pharmaceutically effective substance(s), preferably in the dark and under cooling, more preferably at temperatures below 10 °C, particularly preferable at temperatures in the range of 0 to 5 °C.

23. The bait for animals according to one or more than one of the claims 1 to 11 for use in preventing and/or treating diseases of wildlife animals.

24. The bait for animals according to claim 23 for use in fighting against rabies of foxes.

## Revendications

1. Appât pour animaux, pour la prévention et/ou le traitement de maladies d'animaux vivant à l'état sauvage, contenant une ou plusieurs substance(s) active(s) pharmaceutique(s), composé d'une matière qui enveloppe complètement la ou les substance(s) active(s) pharmaceutique(s), qui n'entre pas en interaction avec elle(s) et qui est en même temps constitué d'une substance attractive spécifique de l'espèce animale à appâter, éventuellement déposée sur le côté extérieur de l'enveloppe complète qui en est formée, y adhère, dans lequel la substance attractive n'est pas un appât carné.

2. Appât pour animaux selon la revendication 1, comprenant en supplément une substance attractive spécifique de l'espèce animale sauvage à appâter sur le côté extérieur de la matière qui enveloppe complètement la ou les substance(s) active(s) pharmaceutique(s).

3. Appât pour animaux selon la revendication 1 ou 2, comprenant en supplément un agent (marqueur) au moyen duquel la prise de l'appât par un animal sauvage peut être contrôlée après son abattage ou sa capture et sa mort, de préférence de la tétracycline ou du bleu de méthylène.

4. Appât pour animaux selon une ou plusieurs des revendications 1 à 3, comprenant, comme substance active pharmaceutique, un vaccin, de préférence un vaccin liquide, dans un mode plus préféré, un vaccin liquide convenant pour la prise orale par les animaux sauvages, et dans un mode encore plus préféré, un vaccin contre la rage.

5. Appât pour animaux selon une ou plusieurs des revendications 1 à 4, comprenant, comme matière qui enveloppe complètement la ou les substance(s) active(s) pharmaceutique(s), une matière qui, sur le côté dirigé vers la ou les substance(s) active(s), est compatible avec elle(s) et qui est résistante à l'humidité, à la chaleur et à la lumière sur son côté extérieur et présente une grande résistance mécanique.

6. Appât pour animaux selon la revendication 5, dans lequel la matière qui enveloppe complètement la ou les substance(s) active(s) pharmaceutique(s) présente un poids par unité de surface compris dans l'intervalle de 50 à 150 g/m².

7. Appât pour animaux selon une ou plusieurs des revendications 1 à 6, dans lequel la matière qui enveloppe complètement la ou les substance(s) active(s) pharmaceutique(s) est une matière composite comprenant du papier et un polymère synthétique, dans un mode particulièrement préféré, une matière composite faite de papier kraft et d'un polyéthylène à basse densité ayant un indice de fusion d'environ 4g/10min et une densité d'environ 0,92 g/cm³.

8. Appât pour animaux selon une ou plusieurs des revendications 1 à 7, qui se présente sous la forme d'un petit paquet de substance active, éventuellement revêtu sur son côté extérieur d'une substance attractive spécifique de l'espèce animale sauvage à appâter, contenant une ou plusieurs substance(s) active(s) pharmaceutique(s) entre deux couches d'une matière composite, et scellé à joint étanche le long de ses bords extérieurs.

9. Appât pour animaux selon la revendication 8, qui présente une forme rectangulaire, de préférence carrée, triangulaire, ronde ou ovale.

10. Appât pour animaux selon une ou plusieurs des revendications 1 à 9, qui présente une épaisseur comprise dans l'intervalle de 1 à 5 mm.

11. Appât pour animaux selon une ou plusieurs des revendications 1 à 10, dans lequel la substance attractive spécifique de l'espèce animale sauvage à appâter comprend de la farine animale, de la farine de viande, de la farine de poisson, et/ou des hydrolysats de ces farines ou des mélanges de ces matières.

12. Procédé de préparation d'un appât contenant une ou plusieurs substance(s) active(s) pharmaceutique(s), pour la prévention et/ou le traitement de maladies d'animaux vivant à l'état sauvage, caractérisé par les phases consistant en ce que
(a) on dispose une matière appropriée pour envelopper complètement la ou les substance(s) active(s) pharmaceutique(s), qui n'entre pas en interaction avec elle(s), et qui est en même temps constituée de manière qu'une substance attractive spécifique de l'espèce animale à appâter, éventuellement déposée sur le côté extérieur de l'enveloppe complète qui en est formée, y adhère et on en forme une poche, la substance attractive n'étant pas un appât carné ;
(b) on remplit la poche avec la ou les substance(s) active(s) pharmaceutique(s) et on la scelle en formant une enveloppe complète dans des conditions qui n'altèrent pas la ou les substance(s) active(s) pharmaceutique(s) ;
(c) on stocke éventuellement le petit paquet de substance(s) active(s) ainsi formé, dans des conditions appropriées ; et
(d) on le disperse.

13. Procédé selon la revendication 12, dans lequel la dispersion de l'appât consiste à le larguer d'un avion.

14. Procédé selon la revendication 12 ou 13, caractérisé par l'étape supplémentaire consistant en ce qu'on revêt le petit paquet de substance active avec une substance attractive spécifique de l'espèce animale à appâter
(a) après l'étape b et avant l'étape c ; ou
(b) après l'étape c et avant l'étape de dispersion d, de préférence immédiatement avant la dispersion, dans un mode particulièrement préféré, immédiatement avant le largage à partir de l'avion.

15. Procédé selon la revendication 14, dans lequel le dépôt de la substance attractive s'effectue en étalant, en déposant au pinceau ou en pulvérisant une substance attractive liquide durcissable sur le petit paquet de substance active ou en plongeant le petit paquet de substance active dans une substance attractive liquide, durcissable.

16. Procédé selon une ou plusieurs des revendications 12 à 15, dans lequel on utilise, comme matière pour envelopper la ou les substance(s) active(s) pharmaceutique(s), une matière qui, sur le côté dirigé vers la ou les substance(s) active(s), est compatible avec elle(s) et qui est résistante à l'humidité, à la chaleur et à la lumière sur son côté extérieur et présente une grande résistance mécanique.

17. Procédé selon une ou plusieurs des revendications 12 à 16, dans lequel on utilise, comme matière pour envelopper la ou les substance(s) active(s) pharmaceutique(s), une matière qui présente un poids par unité de surface compris dans l'intervalle de 50 à 150 g/m².

18. Procédé selon une ou plusieurs des revendications 12 à 17, dans lequel on utilise, comme matière qui enveloppe complètement la ou les substance(s) active(s) pharmaceutique(s), une matière composite comprenant du papier et un polymère synthétique, dans un mode particulièrement préféré, une matière composite faite de papier kraft et d'un polyéthylène à basse densité ayant un indice de fusion d'environ 4 g/10 min et une densité d'environ 0,92 g/cm³.

19. Procédé selon une ou plusieurs des revendications 12 à 18, dans lequel le chargement de la ou des substance(s) active(s) pharmaceutique(s) et la formation d'une enveloppe complète s'effectuent dans des conditions qui ne sont pas préjudiciables à l'activité ni à l'efficacité de la ou des substance(s) active(s) pharmaceutique(s), de préférence dans des conditions stériles et/ou sous réfrigération.

20. Procédé selon une ou plusieurs des revendications 12 à 19, dans lequel on utilise, comme substance active pharmaceutique, un vaccin liquide, de préférence, un vaccin pouvant être absorbé par les animaux sauvages par voie orale et, dans un mode particulièrement préféré, un vaccin liquide contre la rage pouvant être absorbé par voie orale par les animaux sauvages.

21. Procédé selon une ou plusieurs des revendications 12 à 20, dans lequel l'enveloppement complet de la ou des substance(s) active(s) pharmaceutique(s) avec la matière flexible s'effectue par scellement à chaud ponctuel.

22. Procédé selon une ou plusieurs des revendications 12 à 21, dans lequel le stockage s'effectue avec conservation de l'activité et/ou de l'efficacité de la ou des substance(s) active(s) pharmaceutique(s), de préférence dans l'obscurité et sous réfrigération, de préférence à des températures inférieures à 10°C, dans un mode particulièrement préféré, à des températures comprises dans l'intervalle de 0 à 5°C.

23. Appât pour animaux selon une ou plusieurs revendications 1 à 11 pour une utilisation de prévention et/ou de traitement des maladies d'animaux vivant à l'état sauvage.

24. Appât pour animaux selon la revendication 23 pour une utilisation de lutte chimique de la rage pour les renards.
